(19) Europäisches Patentamt
European Patent Office
Office européen des brevets

(11) **EP 1 852 413 A1**

(12) **EUROPÄISCHE PATENTANMELDUNG**

(43) Veröffentlichungstag:
**07.11.2007 Patentblatt 2007/45**

(51) Int Cl.:
*C07C 51/265* (2006.01)    *C07C 63/16* (2006.01)
*B01J 23/22* (2006.01)    *B01J 35/00* (2006.01)

(21) Anmeldenummer: **06008816.8**

(22) Anmeldetag: **27.04.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **BASF AKTIENGESELLSCHAFT**
**67056 Ludwigshafen (DE)**

(72) Erfinder:
• **Lautensack, Thomas**
**69488 Birkenau (DE)**

• **Almann, Hans-Martin**
**74867 Neunkirchen (DE)**
• **Wilmer, Hagen, Dr.**
**67071 Ludwigshafen (DE)**

(74) Vertreter: **Reitstötter - Kinzebach**
**Patentanwälte,**
**Sternwartstrasse 4**
**81679 München (DE)**

(54) **Verfahren zur Gasphasenoxidation unter Verwendung einer Moderatorlage**

(57)     Verfahren zur Gasphasenoxidation, bei dem man einen gasförmigen Strom, der wenigstens einen aromatischen Kohlenwasserstoff und molekularen Sauerstoff umfasst, durch eine oder mehrere Katalysatorlagen leitet, wobei zwischen zwei in Strömungsrichtung des gasförmigen Stroms hintereinander angeordneten Katalysatorlagen eine Moderatorlage angeordnet ist, wobei die Moderatorlage katalytisch weniger aktiv als die stromaufwärts und stromabwärts angrenzenden Katalysatoren oder katalytisch inaktiv ist. Die gewünschten Oxidationsprodukte werden über längere Zeiträume in hoher Ausbeute erhalten.

Fig. 2

EP 1 852 413 A1

**Beschreibung**

Die vorliegende Erfindung betrifft ein Verfahren zur Gasphasenoxidation, bei dem man einen gasförmigen Strom, der einen aromatischen Kohlenwasserstoff und molekularen Sauerstoff umfasst, durch zwei oder mehrere Katalysatorlagen leitet.

Eine Vielzahl von Carbonsäuren und/oder Carbonsäureanhydriden wird technisch durch die katalytische Gasphasenoxidation von aromatischenKohlenwasserstoffen, wie Benzol, den Xylolen, Naphthalin, Toluol oder Durol, in Festbettreaktoren hergestellt. Man kann auf diese Weise z. B. Benzoesäure, Maleinsäureanhydrid, Phthalsäureanhydrid, Isophthalsäure, Terephthalsäure oder Pyromellithsäureanhydrid erhalten. Im Allgemeinen leitet man ein Gemisch aus einem sauerstoffhaltigen Gas und dem zu oxidierenden Ausgangsmaterial durch Rohre, in denen sich eine Schüttung eines Katalysators befindet. Zur Temperaturregelung sind die Rohre von einem Wärmeträgermedium, beispielsweise einer Salzschmelze, umgeben.

Obgleich die überschüssige Reaktionswärme durch das Wärmeträgermedium abgeführt wird, kann es in der Katalysatorschüttung zur Ausbildung lokaler Temperaturmaxima (Hot Spots) kommen, in denen eine höhere Temperatur herrscht als im übrigen Teil der Katalysatorschüttung. Diese Hot Spots führen zu Nebenreaktionen, wie der Totalverbrennung des Ausgangsmaterials, oder zur Bildung unerwünschter, vom Reaktionsprodukt nicht oder nur mit großem Aufwand abtrennbarer Nebenprodukte.

Außerdem kann der Katalysator ab einer bestimmten Hot Spot-Temperatur irreversibel geschädigt werden. Daher muss beim Anfahren des Verfahrens die Beladung des gasförmigen Stroms mit dem zu oxidierenden Kohlenwasserstoff anfangs sehr niedrig gehalten werden und kann nur langsam gesteigert werden. Der endgültige Produktionszustand wird oft erst nach einigen Wochen erreicht.

Zur Abschwächung dieser Hot Spots wurden verschiedene Maßnahmen getroffen. Insbesondere wurde, wie in der DE-A 40 13 051 beschrieben, dazu übergegangen, unterschiedlich aktive Katalysatoren schichtweise in der Katalysatorschüttung anzuordnen, wobei in der Regel der weniger aktive Katalysator zum Gaseintritt hin und der aktivere Katalysator zum Gasaustritt hin gelegen ist.

Die DE 198 23 262 beschreibt ein Verfahren zur Herstellung von Phthalsäureanhydrid mit mindestens drei in Lagen übereinander angeordneten Schalenkatalysatoren, wobei die Katalysatoraktivität von Schicht zu Schicht von der Gaseintrittsseite zur Gasaustrittsseite ansteigt.

Die EP-A 1 063 222 beschreibt ein Verfahren zur Herstellung von Phthalsäureanhydrid, welches in einem oder mehreren Festbettreaktoren durchgeführt wird. Die Katalysatorbetten in den Reaktoren weisen drei oder mehr als drei individuelle, im Reaktor aufeinander folgende Katalysatorlagen auf. Nach Durchgang durch die erste Katalysatorlage unter den Reaktionsbedingungen sind 30 bis 70 Gew.-% des eingesetzten o-Xylol, Naphthalins oder des Gemisch aus beiden umgesetzt. Nach der zweiten Lage sind 70 Gew.-% oder mehr umgesetzt.

Die EP 326 536 beschreibt ein Verfahren zur Herstellung von Maleinsäureanhydrid durch Gasphasenoxidation geeigneter nichtaromatischer Kohlenwasserstoff an einem Phosphor-Vanadium-Mischoxidkatalysator. Durch Mischen des Katalysators mit einem inerten festen Material wird der Ausbeuteabfall pro Monat bei langfristigem Betrieb vermindert.

Die WO 2005/063673 beschreibt ein Verfahren zur Herstellung ungesättigter Aldehyde und/oder Carbonsäuren durch Partialoxidation an einem Katalysatorfestbett, wobei in der Reaktionszone eine Lage inaktives Material an der Stelle eingeschoben ist, an der die Lage des HotSpots erwartet wird. Der stromabwärts und stromaufwärts zur inaktiven Materiallage angeordnete Katalysator ist identisch.

Die Aktivität der zur Gasphasenoxidation eingesetzten Katalysatoren bzw. Katalysatorsysteme nimmt mit zunehmender Betriebsdauer ab. Es gelangt ein höherer Anteil nicht umgesetzter Kohlenwasserstoffe oder teiloxidierter Intermediate in weiter stromabwärts gelegene Bereiche der Katalysatorschüttung. Die Reaktion verlagert sich zunehmend zum Reaktorausgang und der Hot Spot wandert stromabwärts. Man kann der Katalysatordeaktivierung in begrenztem Umfang durch Erhöhung der Temperatur des Wärmeträgermediums entgegenwirken. Die Erhöhung der Temperatur des Wärmeträgermediums und/oder die Verschiebung des Hot Spots führen bei mehrlagigen Katalysatorsystemen dazu, dass die Temperatur, mit der das Gasgemisch in eine nachgelagerte Katalysatorlage eintritt, steigt. Da nachgelagerte Katalysatorlagen in der Regel zwar aktiver aber weniger selektiv sind, nehmen unerwünschte Überoxidation und andere Nebenreaktionen zu. Insgesamt sinkt mit der Betriebsdauer die Produktausbeute bzw. Selektivität.

Der Erfindung liegt die Aufgabe zu Grunde, ein Verfahren bereitzustellen, das es gestattet, die gewünschten Oxidationsprodukte über längere Zeiträume in hoher Ausbeute zu erhalten.

Die Aufgabe wird gelöst durch ein Verfahren zur Gasphasenoxidation, bei dem man einen gasförmigen Strom, der wenigstens einen aromatischen Kohlenwasserstoff und molekularen Sauerstoff umfasst, durch wenigstens zwei in Strömungsrichtung des gasförmigen Stroms hintereinander angeordnete Katalysatorlagen leitet, wobei die Aktivität der Katalysatoren in aneinandergrenzenden Katalysatorlagen voneinander verschieden ist, dadurch gekennzeichnet, dass zwischen zwei in Strömungsrichtung des gasförmigen Stroms hintereinander angeordneten Katalysatorlagen eine Moderatorlage angeordnet ist, wobei die Moderatorlage katalytisch weniger aktiv als die stromaufwärts und stromabwärts angrenzenden Katalysatoren oder katalytisch inaktiv ist.

Als Katalysatorlage wird vorliegend die Schüttung eines Katalysators mit im Wesentlichen einheitlicher Aktivität angesehen, d. h. mit im Wesentlichen einheitlicher Zusammensetzung der Aktivmasse, Aktivmasseanteil und Packungsdichte (abgesehen von unvermeidlichen Fluktuationen beim Befüllen des Reaktors). Aufeinanderfolgende Katalysatorlagen unterscheiden sich somit in der Aktivität der enthaltenen Katalysatoren. Dem Fachmann sind verschiedene Maßnahmen zur Steuerung der Katalysatoraktivität bekannt, wie weiter unten ausgeführt. Innerhalb einer Katalysatorlage sind die Katalysatorteilchen nicht durch Teilchen eines inerten Materials verdünnt.

Unter Aktivität eines Katalysators bzw. einer Katalysatorlage wird der Umsatz verstanden, der unter identischen Bedingungen (insbesondere hinsichtlich Katalysatorvolumen, volumenbezogener Raumgeschwindigkeit (gas hourly space velocity GHSV) bzw. Luftmenge, Temperatur des Wärmeträgermediums, Kohlenwasserstoff-Beladung des gasförmigen Stroms) in einer Testanlage gemessen wird. Je höher der Umsatz eines Katalysators bzw. einer Katalysatorlage desto höher ist dessen/deren Aktivität. Diese Methode ist insbesondere zum Vergleich von Aktivitäten bzw. zur Bestimmung relativer Katalysatoraktivitäten geeignet.

Die Erfindung sieht eine oder mehrere Moderatorlagen vor, die so zwischen zwei Katalysatorschüttungen angeordnet sind, dass sie vom gasförmigen Strom, der die stromaufwärts gelegene Katalysatorschüttung verlässt, passiert wird, bevor dieser in die stromabwärts gelegene Katalysatorschüttung eintritt.

Die Moderatorlage besteht zweckmäßigerweise aus einer Schüttung eines teilchenförmigen Materials. Im Hinblick auf ein leichtes Befüllen des Reaktors und einen gleichmäßigen Druckverlust weist das teilchenförmige Material zweckmäßigerweise ähnliche Dimensionen auf wie die Katalysatorteilchen.

Die Moderatorlage kann katalytisch inaktiv sein. In diesem Fall besteht sie aus einem inerten Material, wie es z. B. auch als Katalysatorträger verwendet wird. Geeignete Trägermaterialien sind beispielsweise Quarz ($SiO_2$), Porzellan, Magnesiumoxid, Zinndioxid, Siliciumcarbid, Rutil, Tonerde ($Al_2O_3$), Aluminiumsilikat, Steatit (Magnesiumsilikat), Zirkoniumsilikat, Cersilikat oder Mischungen dieser Trägermaterialien. Die Moderatorlage kann auch Gewebe, Gestricke oder Gewirke aus Fasern oder Metalldrähten umfassen.

Die Moderatorlage kann auch katalytische Aktivität aufweisen. In diesem Fall ist die Moderatorlage sowohl weniger katalytisch aktiv als die stromaufwärts gelegene Katalysatorschüttung als auch weniger katalytisch aktiv als die stromabwärts gelegene Katalysatorschüttung. Dies kann durch einen hohen Gehalt deaktivierender Zusätze, geringen Aktivmasseanteil, Verdünnung eines Katalysators mit inertem Material und/oder andere Maßnahmen, die dem Fachmann geläufig sind, erreicht werden.

Die Moderatorlage ist zwischen zwei aufeinander folgenden Katalysatorlagen angeordnet, wobei die stromaufwärts gelegene Katalysatorschüttung und die stromabwärts gelegene Katalysatorschüttung unterschiedliche Aktivität aufweisen. In der Regel weist die stromabwärts zur Moderatorlage gelegene Katalysatorschüttung eine höhere Aktivität auf als die stromaufwärts zur Moderatorlage gelegene Katalysatorschüttung. Das Verhältnis des Volumens der Moderatorlage zum Volumen der stromaufwärts zur Moderatorlage gelegenen Katalysatorlage beträgt vorzugsweise 0,05 bis 0,35, insbesondere 0,1 bis 0,25.

Der Anteil des Volumens der Moderatorlagen am Gesamtvolumen von Katalysator- und Moderatorlagen beträgt im Allgemeinen nicht mehr als 40 %.

In bevorzugten Ausführungsformen leitet man den gasförmigen Strom durch wenigstens drei in Strömungsrichtung des gasförmigen Stroms hintereinander angeordnete Katalysatorlagen, z. B. durch drei, vier oder fünf Katalysatorlagen.

Wenn mehrere Katalysatorlagen zum Einsatz kommen, sind verschiedene Konfigurationen der Aktivitätsabstufung möglich. In einer bevorzugten Ausführungsform nimmt die Aktivität der Katalysatoren in Strömungsrichtung des gasförmigen Stroms von der am nächsten zum Gaseinritt gelegenen Katalysatorlage zu der am nächsten zum Gasaustritt gelegenen Katalysatorlage von einer Katalysatorlage zur nächsten stetig zu. Dabei ist vorzugsweise zumindest zwischen der in Strömungsrichtung vorletzten und letzten Katalysatorlage eine Moderatorlage angeordnet.

In einer anderen bevorzugten Ausführungsform leitet man den gasförmigen Strom durch mehr als drei in Strömungsrichtung des gasförmigen Stroms hintereinander angeordnete Katalysatorlagen; dabei nimmt die Aktivität der Katalysatoren über eine Abfolge von wenigstens drei der Katalysatorlagen in Strömungsrichtung des gasförmigen Stroms von einer Katalysatorlage zur nächsten zu. So ist es möglich, als am meisten stromaufwärts gelegene Katalysatorlage eine relativ kurze, hochaktive Katalysatorlage vorzusehen, an die sich stromabwärts eine weniger aktive Katalysatorlage anschließt, wobei sich an diese weniger aktive Katalysatorlage weitere Lagen mit stufenweise steigender Aktivität anschließen können.

Die erfindungsgemäß vorgesehene(n) Moderatorlage(n) bewirkt (bewirken) eine Abkühlung des gasförmigen Stroms, bevor dieser in die Schüttung des stromabwärts zur Moderatorlage angeordneten Katalysators eintritt. Die Abkühlung ist aus dem Temperaturprofil, d. h. einer Auftragung der Temperatur in Abhängigkeit von der Katalysatorbettlänge, ersichtlich. Das Temperaturprofil kann mittels Thermoelementen, die in Thermorohren in verschiedenen Höhen, z. B. äquidistant, angeordnet sind, oder mit einem in der Höhe verschiebbaren Thermoelement leicht bestimmt werden.

Vorzugsweise ist die Temperatur an der stromabwärtigen Grenze einer Moderatorlage um wenigstens 2 K, insbesondere um wenigstens 4 K, niedriger als der aus dem stromaufwärts zur Moderatorlage gelegenen Katalysator extrapolierte Temperaturverlauf. Der extrapolierte Temperaturverlauf kann durch die Gleichung

$$(\partial T \cdot x)$$

berechnet werden, worin aT für die Steigung des Temperaturprofils an der Grenze des stromaufwärts zur Moderatorlage gelegenen Katalysators zur Moderatorlage steht und x für die Länge der Moderatorlage in Strömungsrichtung steht. Die Steigung des Temperaturprofils kann durch Anlegen einer Tangente an das Temperaturprofil an der Grenze des stromaufwärts zur Moderatorlage gelegenen Katalysators ermittelt werden.

Bevorzugt beträgt der Temperaturabfall über die Moderatorlage wenigstens 4 K, insbesondere wenigstens 6 K, und besonders bevorzugt wenigstens 8 K. Als Temperaturabfall wird hier die aus dem Temperaturprofil ermittelbare Differenz zwischen der Temperatur an der stromaufwärtigen und der stromabwärtigen Grenze der Moderatorlage betrachtet.

Das erfindungsgemäße Verfahren eignet sich besonders zur Gasphasenoxidation aromatischer $C_6$- bis $C_{10}$-Kohlenwasserstoffe, wie Benzol, den Xylolen, Toluol, Naphthalin oder Durol (1,2,4,5-Tetramethylbenzol) zu Carbonsäuren und/oder Carbonsäureanhydriden wie Maleinsäureanhydrid, Phthalsäureanhydrid, Benzoesäure und/oder Pyromellithsäuredianhyrid. Das Verfahren eignet sich besonders zur Herstellung von Phthalsäureanhydrid aus o-Xylol und/oder Naphthalin.

Bevorzugt umfasst die katalytisch aktive Masse aller Katalysatoren zumindest Vanadiumoxid und Titandioxid. Maßnahmen zur Steuerung der Aktivität von Gasphasenoxidationskatalysatoren auf Basis von Vanadiumoxid und Titandioxid sind dem Fachmann an sich bekannt.

So können in der katalytisch aktiven Masse Verbindungen enthalten sein, die als Promotoren die Aktivität und Selektivität des Katalysators beeinflussen.

Als aktivitäts- bzw. selektivitätsbeeinflussende Faktoren seien beispielhaft Alkalimetallverbindungen, insbesondere Cäsiumoxid, Lithium-, Kalium-, Natrium- und Rubidiumoxid, sowie phosphor- oder schwefelhaltige Verbindungen genannt.

Eine weitere Möglichkeit der Aktivitätssteuerung besteht in der Variation des Anteils der Aktivmasse oder des $V_2O_5$-Gehalts am Gesamtgewicht des Katalysators, wobei höhere Aktivmassen- oder $V_2O_5$-Gehalte eine höhere Aktivität bedingen und umgekehrt.

Bei den im erfindungsgemäßen Verfahren verwendeten Katalysatoren handelt es sich im Allgemeinen um Schalenkatalysatoren, bei denen die katalytisch aktive Masse schalenförmig auf einem inerten Träger aufgebracht ist. Die Schichtdicke der katalytisch aktiven Masse beträgt in der Regel 0,02 bis 0,2 mm, vorzugsweise 0,05 bis 0,1 mm. Im Allgemeinen weisen die Katalysatoren eine schalenförmig aufgebrachte Aktivmasseschicht auf einem inerten Träger auf.

Als inertes Trägermaterial können praktisch alle Trägermaterialien des Standes der Technik, wie sie vorteilhaft bei der Herstellung von Schalenkatalysatoren für die Oxidation aromatischer Kohlenwasserstoffe zu Aldehyden, Carbonsäuren und/oder Carbonsäureanhydriden eingesetzt werden, Verwendung finden, beispielsweise Quarz ($SiO_2$), Porzellan, Magnesiumoxid, Zinndioxid, Siliciumcarbid, Rutil, Tonerde ($Al_2O_3$), Aluminiumsilikat, Steatit (Magnesiumsilikat), Zirkoniumsilikat, Cersilikat oder Mischungen dieser Trägermaterialien. Das Trägermaterial ist in der Regel nicht-porös. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Bevorzugt wird Steatit in Form von Kugeln mit einem Durchmesser von 3 bis 6 mm oder von Ringen mit einem äußeren Durchmesser von 5 bis 9 mm und einer Länge von 4 bis 7 mm verwendet.

Die Aufbringung der einzelnen Schichten des Schalenkatalysators kann mit beliebigen an sich bekannten Methoden erfolgen, z. B. durch Aufsprühen von Lösungen oder Suspensionen in der Dragiertrommel oder Beschichtung mit einer Lösung oder Suspension in einer Wirbelschicht. Dabei können der katalytisch aktiven Masse organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat, Vinylacetat/Ethylen sowie Hydroxyethylcellulose zugesetzt werden, wobei mit Vorteil Bindermengen von 3 bis 20 Gew.-%, bezogen auf den Feststoffgehalt der Lösung der Aktivmassenbestandteile, eingesetzt werden. Wird die katalytisch aktive Masse ohne organische Bindemittel auf den Träger aufgetragen, so sind Beschichtungstemperaturen über 150°C von Vorteil. Bei Zusatz der oben angegebenen Bindemittel liegen die brauchbaren Beschichtungstemperaturen je nach verwendetem Bindemittel zwischen 50 und 450 °C. Die aufgetragenen Bindemittel brennen nach dem Einfüllen des Katalysators und Inbetriebnahme des Reaktors innerhalb kurzer Zeit aus. Der Binderzusatz hat zudem den Vorteil, dass die Aktivmasse gut auf dem Träger haftet, so dass Transport und Einfüllen des Katalysators erleichtert werden.

In einer bevorzugten Ausführungsform des erfindungsgemäßen Verfahrens mit drei Katalysatorlagen weisen die Katalysatoren folgende Zusammensetzung auf (wobei die erste Lage die in Strömungsrichtung des Gasstroms am weitesten stromaufwärts angeordnete Lage ist):

für die erste Lage:

7 bis 10 Gew.-% Aktivmasse bezogen auf den gesamten Katalysator, wobei diese Aktivmasse:
6 bis 11 Gew.-% Vanadiumpentoxid
1,2 bis 3 Gew.-% Antimontrioxid
0,1 bis 1 Gew.-% eines Alkali (berechnet als Alkalimetall), insbesondere Cäsiumoxid enthält und als Rest auf 100 Gew.-% Titandioxid, vorzugsweise in Anatasmodifikation mit einer BET-Oberfläche von 5 bis 30 m$^2$/g

für die zweite Lage:

7 bis 12 Gew.-% Aktivmasse bezogen auf den gesamten Katalysator, wobei diese Aktivmasse:
5 bis 13 Gew.-% Vanadiumpentoxid
0 bis 3 Gew.-% Antimontrioxid
0 bis 0,4 Gew.-% eines Alkali (berechnet als Alkalimetall), insbesondere Cäsiumoxid
0 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 10 bis 40 m$^2$/g

für die dritte Lage:

8 bis 12 Gew.-% Aktivmasse bezogen auf den gesamten Katalysator, wobei diese Aktivmasse:
5 bis 30 Gew.-% Vanadiumpentoxid
0 bis 3 Gew.-% Antimontrioxid
0 bis 0,3 Gew.-% eines Alkali (berechnet als Alkalimetall), insbesondere Cäsiumoxid 0,05 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 15 bis 50 m$^2$/g.

Das Verhältnis der von der ersten, zweiten und dritten Lage eingenommenen Volumina beträgt vorzugsweise 100 bis 200 : 40 bis 100 : 40 bis 100.

In einer bevorzugten Ausführungsform des erfindungsgemäßen Verfahrens mit vier Katalysatorlagen weisen die Katalysatoren folgende Zusammensetzung auf (wobei die erste Lage die in Strömungsrichtung des Gasstroms am weitesten stromaufwärts angeordnete Lage ist):

für die erste Lage:

7 bis 10 Gew.-% Aktivmasse bezogen auf den gesamten Katalysator, wobei diese Aktivmasse:
6 bis 11 Gew.-% Vanadiumpentoxid
1,2 bis 3 Gew.-% Antimontrioxid
0,1 bis 1 Gew.-% eines Alkali (berechnet als Alkalimetall), insbesondere Cäsiumoxid

enthält und als Rest zu 100 Gew.-% Titandioxid, vorzugsweise in Anatasmodifikation mit einer BET-Oberfläche von 5 bis 30 m$^2$/g

für die zweite Lage:

7 bis 10 Gew.-% Aktivmasse bezogen auf den gesamten Katalysator, wobei diese Aktivmasse:
4 bis 15 Gew.-% Vanadiumpentoxid
0 bis 3 Gew.-% Antimontrioxid
0,1 bis 1 Gew.-% eines Alkali (berechnet als Alkalimetall), insbesondere Cäsiumoxid
0 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 10 bis 35 m$^2$/g

für die dritte Lage:

7 bis 10 Gew.-% Aktivmasse bezogen auf den gesamten Katalysator, wobei diese Aktivmasse:
5 bis 13 Gew.-% Vanadiumpentoxid
0 bis 3 Gew.-% Antimontrioxid
0 bis 0,4 Gew.-% eines Alkali (berechnet als Alkalimetall), insbesondere Cäsiumoxid
0 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 15 bis 40 m$^2$/g

für die vierte Lage:

8 bis 12 Gew.-% Aktivmasse bezogen auf den gesamten Katalysator, wobei diese Aktivmasse:
10 bis 30 Gew.-% Vanadiumpentoxid
0 bis 3 Gew.-% Antimontrioxid
0,05 bis 0,4 Gew.-% Phosphorpentoxid (berechnet als P)

enthält und als Rest zu 100 Gew.-% Titandioxid, vorzugsweise in Anatasmodifikation mit einer BET-Oberfläche von 15 bis 50 m$^2$/g.

Das Verhältnis der von der ersten, zweiten, dritten und vierten Lage eingenommenen Volumina beträgt vorzugsweise 80 bis 160 : 30 bis 100 : 30 bis 100 : 30 bis 100.

Gewünschtenfalls kann man für die Phthalsäureanhydridherstellung noch einen nachgeschalteten Finishing-Reaktor vorsehen, wie er beispielsweise in der DE-A 198 07 018 oder DE-A 20 05 969 A beschrieben ist. Als Katalysator verwendet man dabei im Vergleich zum Katalysator der letzten Lage vorzugsweise einen noch aktiveren Katalysator.

Die Katalysatoren werden üblicherweise in Reaktionsrohre gefüllt, die in von außen auf die Reaktionstemperatur, beispielsweise mit einem Wärmeträgermedium, z. B. einer Salzschmelze, thermostatisiert sind. Über die so bereitete Katalysatorschüttung wirdder gasförmige Strom bei Temperaturen von im allgemeinen 300 bis 450 °C, vorzugsweise von 320 bis 420 °C und besonders bevorzugt von 340 bis 400 °C und bei einem Überdruck von im allgemeinen 0,1 bis 2,5 bar, vorzugsweise von 0,3 bis 1,5 bar mit einer Raumgeschwindigkeit von im allgemeinen 750 bis 5000 $h^{-1}$ geleitet.

Das dem Katalysator zugeführte Reaktionsgas wird im allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit dem zu oxidierenden, aromatischen Kohlenwasserstoff erzeugt, wobei das molekularen Sauerstoff enthaltende Gas im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten kann. Zur Erzeugung des Reaktionsgases wird das molekularen Sauerstoff enthaltende Gas im allgemeinen mit 30 g bis 150 g je $Nm^3$ Gas, vorzugsweise 60 bis 120 g je $Nm^3$, des zu oxidierenden Kohlenwasserstoffs beschickt.

Man kann zwei oder mehr Zonen, vorzugsweise zwei Zonen der im Reaktionsrohr befindlichen Katalysatorschüttung auf unterschiedliche Reaktionstemperaturen thermostatisieren, wozu beispielsweise Reaktoren mit getrennten Salzbädern eingesetzt werden können. Alternativ kann die Gasphasenoxidation auch ohne Aufteilung in Temperaturzonen bei einer Reaktionstemperatur durchgeführt werden.

Die Erfindung wird durch die beigefügte Figur und die folgenden Beispiele näher veranschaulicht.

Fig. 1 zeigt das Temperaturprofil eines zweilagigen Katalysatorsystems bei der Gasphasenoxidation von o-Xylol zu Phthalsäureanhydrid (ohne Moderatorlage).

Fig. 2 zeigt das Temperaturprofil eines zweilagigen Katalysatorsystems bei der Gasphasenoxidation von o-Xylol zu Phthalsäureanhydrid mit katalytisch inaktiver Moderatorlage zwischen erster und zweiter Katalysatorlage.

Beispiele

Vergleichsbeispiel

Man verwendete ein käufliches zweilagiges Katalysatorsystem, das aus zwei Katalysatoren unterschiedlicher Aktivität bestand. Der stromaufwärts gelegene Katalysator mit niedrigerer Aktivität wird als Selektivkatalysator, der stromabwärts gelegene Katalysator als Aktivkatalysator bezeichnet. Die Aktivmasse beider Katalysatoren enthielt Titandioxid und Vanadiumoxid. Die Aktivmasse war auf Steatitträger aufgebracht.

Von unten nach oben wurden in einen Reaktor, bestehend aus einem Eisenrohr mit einer lichten Weite von 25 mm und einer Länge von 360 cm, 130 cm Aktivkatalysator und 170 cm Selektivkatalysator eingefüllt. Das Eisenrohr war zur Temperaturregelung von einer Salzschmelze einer Temperatur von 350 °C umgeben. Durch das Rohr wurden stündlich von oben nach unten 4 $Nm^3$ Luft pro Rohr mit Beladungen an 98,5gew.-% igem o-Xylol von 80 g/$Nm^3$ Luft geleitet.

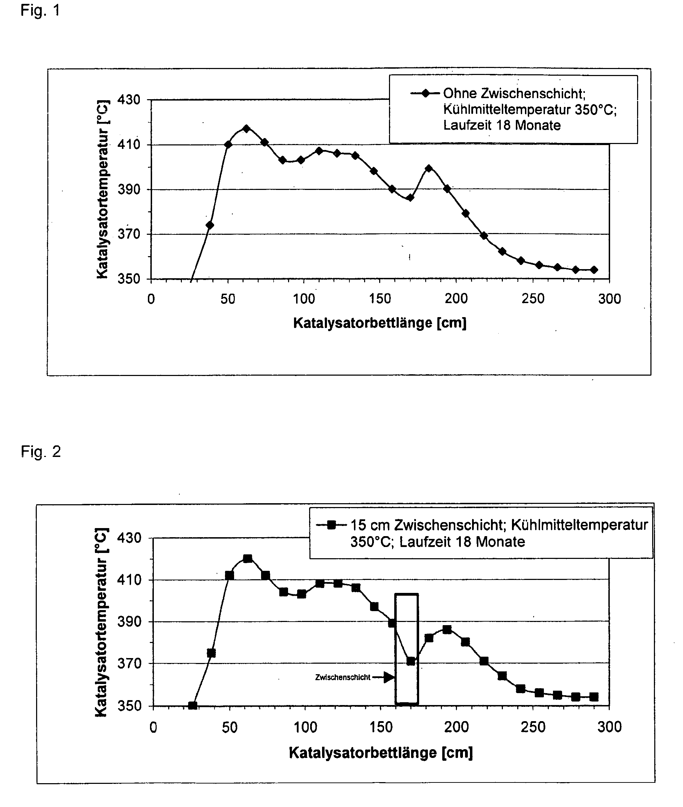

Das Temperaturprofil dieses Katalysatorsystems nach 18 Monaten Betriebszeit ist in Figur 1 dargestellt. Man erkennt, dass der gasförmige Strom mit einer Temperatur von etwa 385 °C in die Lage des Aktivkatalysators eintritt. Die Ausbeute betrug nach dieser Betriebszeit 113,5 kg Phthalsäureanhydrid pro 100 kg eingesetztem o-Xylol (bezogen auf reines o-Xylol).

Erfindungsgemäßes Beispiel

Das Vergleichsbeispiel wurde wiederholt, wobei jedoch die Schüttungslängen des Katalysatorsystems zugunsten einer Moderatorlage modifiziert wurden. Die Moderatorlage bestand aus Steatitringen (7 mm Aussendurchmesser, 7mm Höhe, 4 mm Innendurchmesser). Man füllte nacheinander 125 cm Aktivkatalysator, 15 cm Moderatorlage und 170 cm Selektivkatalysator in die Rohre.

Das Temperaturprofil dieses Katalysatorsystems nach 18 Monaten Betriebszeit bei einer Salzbadtemperatur von 350 °C ist in Figur 2 dargestellt. Man erkennt, dass der gasförmige Strom mit einer Temperatur von etwa 368 °C in die Lage des Aktivkatalysators eintritt, d. h. mit einer gegenüber dem Vergleichsbeispiel um etwa 17 °C tieferen Temperatur. Die Ausbeute betrug nach dieser Betriebszeit 114,3 kg Phthalsäureanhydrid pro 100 kg eingesetztem o-Xylol (bezogen auf reines o-Xylol).

**Patentansprüche**

1. Verfahren zur Gasphasenoxidation, bei dem man einen gasförmigen Strom, der wenigstens einen aromatischen Kohlenwasserstoff und molekularen Sauerstoff umfasst, durch wenigstens zwei in Strömungsrichtung des gasförmigen Stroms hintereinander angeordnete Katalysatorlagen leitet, wobei die Aktivität der Katalysatoren in aneinandergrenzenden Katalysatorlagen voneinander verschieden ist, **dadurch gekennzeichnet, dass** zwischen zwei in Strömungsrichtung des gasförmigen Stroms hintereinander angeordneten Katalysatorlagen eine Moderatorlage angeordnet ist, wobei die Moderatorlage katalytisch weniger aktiv als die stromaufwärts und stromabwärts angrenzenden Katalysatoren oder katalytisch inaktiv ist.

2. Verfahren nach Anspruch 1, wobei man den gasförmigen Strom durch wenigstens drei in Strömungsrichtung des gasförmigen Stroms hintereinander angeordnete Katalysatorlagen leitet.

3. Verfahren nach Anspruch 1 oder 2, wobei die Aktivität der Katalysatoren in Strömungsrichtung des gasförmigen Stroms von der am nächsten zum Gaseinritt gelegenen Katalysatorlage zu der am nächsten zum Gasaustritt gelegenen Katalysatorlage von einer Katalysatorlage zur nächsten stetig zunimmt.

4. Verfahren nach Anspruch 2, wobei man den gasförmigen Strom durch mehr als drei in Strömungsrichtung des gasförmigen Stroms hintereinander angeordnete Katalysatorlagen leitet und die Aktivität der Katalysatoren über eine Abfolge von wenigstens drei der Katalysatorlagen in Strömungsrichtung des gasförmigen Stroms von einer Katalysatorlage zur nächsten zunimmt.

5. Verfahren nach Anspruch 3 oder 4, wobei zumindest stromaufwärts zur aktivsten Katalysatorlage eine Moderatorlage angeordnet ist.

6. Verfahren nach einem der vorhergehenden Ansprüche, wobei das Verhältnis des Volumens der Moderatorlage zum Volumen der stromaufwärts zur Moderatorlage gelegenen Katalysatorlage 0,02 bis 0,35 beträgt.

7. Verfahren nach einem der vorhergehenden Ansprüche, wobei die Temperatur an der stromabwärtigen Grenze der Moderatorlage um wenigstens 2 K niedriger ist als

$$(\partial T \cdot x)$$

worin $\partial T$ für die Steigung des Temperaturprofils an der Grenze des stromaufwärts zur Moderatorlage gelegenen Katalysators zur Moderatorlage steht und x für die Länge der Moderatorlage in Strömungsrichtung steht.

8. Verfahren nach einem der vorhergehenden Ansprüche, wobei der Temperaturabfall über die Moderatorlage wenigstens 4 K beträgt.

9. Verfahren nach einem der vorhergehenden Ansprüche, bei dem die katalytisch aktive Masse aller Katalysatoren zumindest Vanadiumoxid und Titandioxid umfasst.

10. Verfahren nach einem der vorhergehenden Ansprüche, bei dem man als Kohlenwasserstoff o-Xylol und/oder Naphthalin einsetzt und Phthalsäureanhydrid herstellt.

Fig. 1

Fig. 2

**Europäisches Patentamt** EUROPÄISCHER RECHERCHENBERICHT

Nummer der Anmeldung

EP 06 00 8816

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,X | WO 2005/063673 A (LG CHEMICAL LTD [KR]) 14. Juli 2005 (2005-07-14) <br> * Seite 7, Zeilen 8-19,22-28 * <br> * Seite 9, Zeilen 8-13 * <br> * Seite 10, Zeilen 16-24 * <br> * Seite 11, Zeilen 11-19 * <br> * Anspruch 3 * <br> ----- | 1-10 | INV. <br> C07C51/265 <br> C07C63/16 <br> B01J23/22 <br> B01J35/00 |
| E | WO 2006/092305 A (SUED CHEMIE AG [DE]; GUECKEL CHRISTIAN [US]; DIALER HARALD [DE]; ESTEN) 8. September 2006 (2006-09-08) <br> * Seite 6: Paragraph a) und b)* <br> * Seite 6, letzten 3 Zeilen bis Seite 7, Zeile10 * <br> * Seite 12, Zeilen 7-9 * <br> * Seiten 9-10 * <br> * Seite 12, Zeilen 7-9 * <br> * Seite 14 * <br> ----- | 1-10 | |

| | | | RECHERCHIERTE SACHGEBIETE (IPC) |
|---|---|---|---|
| | | | C07C <br> B01J |

Der vorliegende Recherchenbericht wurde für alle Patentansprüche erstellt

| Recherchenort | Abschlußdatum der Recherche | Prüfer |
|---|---|---|
| München | 7. November 2006 | Goetz, Gerhard |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 06 00 8816

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-11-2006

| Im Recherchenbericht angeführtes Patentdokument | Datum der Veröffentlichung | Mitglied(er) der Patentfamilie | Datum der Veröffentlichung |
|---|---|---|---|
| WO 2005063673 A | 14-07-2005 | EP 1697294 A1<br>US 2005209484 A1 | 06-09-2006<br>22-09-2005 |
| WO 2006092305 A | 08-09-2006 | KEINE | |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4013051 A
- DE 19823262
- EP 1063222 A
- EP 326536 A
- WO 2005063673 A
- DE 19807018 A
- DE 2005969 A